# EUROPEAN PATENT SPECIFICATION

(11) **EP 2 616 058 B1**
(45) Date of publication and mention of the grant of the patent: **12.08.2015**
(21) Application number: 11826086.8
(22) Date of filing: 19.09.2011
(51) Int. Cl.: A61K 31/192, A61K 31/19, A23L 1/30

(54) **ANIMAL FEED COMPOSITIONS OF ABSCISIC ACID**
TIERFUTTERZUSAMMENSETZUNGEN AUS ABSCISINSÄURE
COMPOSITIONS D'ACIDE ABSCISSIQUE POUR L'ALIMENTATION ANIMALE

(30) Priority: 17.09.2010 US 384020 P
(43) Date of publication of application: 24.07.2013
(73) Proprietor: Valent Biosciences Corporation, Liberty, Illinois 60048 (US)
(72) Inventor: HERRERO, Maria, Pilar, Libertyville, IL 60048 (US); SHAFER, Warren, E., Libertyville, IL 60048 (US)
(74) Representative: Kingsbury, Oliver William
(86) International application number: PCT/US2011/052168
(87) International publication number: WO 2012/037561

(56) References cited:
- US-A1- 2007 184 060
- US-A1- 2008 242 635

## Description

### FIELD OF THE INVENTION

The present invention generally relates to methods for administration of compositions comprising abscisic acid and/or salts thereof to livestock and in aquaculture.

### BACKGROUND OF THE INVENTION

Abscisic acid is a naturally occurring plant hormone that is well known to be involved in plant response to stress. The chemistry and physiology of abscisic acid and its analogs is described by Milborrow, Ann. Rev. Plant Physiol. 1974, 25, 259-307. The naturally occurring enantiomeric form of abscisic acid is (S)-(+)-abscisic acid. The stereochemistry of the side chain of the major part of naturally occurring abscisic acid is 2*-cis-,*4*-trans-,* since that is the isomer that is produced biosynthetically by all green plants and some microorganisms. Abscisic acid is a carboxylic acid, and thus in a medium having an acidic pH, it is protonated in its neutral undissociated form. This uncharged, undissociated form is more lipophilic than a salt of abscisic acid is in its dissociated form of abscisic acid that is present at higher pH (Blumenfeld and Bukovac 1972, Planta 107: 261-268).

Commercial formulations comprising abscisic acid are used in agriculture and horticulture on or around crops and plants for various purposes, such as improving stress tolerance, slowing the growth rate, adjusting flowering phase, and other purposes. Abscisic acid has also been reported to possess insect inhibition qualities. See U.S. Patent Nos. 4,434,180 and 4,209,530. Others have reported potential medicinal properties of abscisic acid, for example U.S. Patent Application No. 2007/184060 discloses a method of using abscisic acid to treat and prevent diseases and disorders, U.S. Patent Application No. 2006/0292215 discloses methods of using abscisic acid for anti-cancer purposes, and International Application No. WO 2007/042983 discloses anti-inflammatory activity of abscisic acid. Abscisic acid has also been used in the field of animal feed, for example, U.S. Patent Application No. 2008/0242635 discloses that the seasonal mobility period of fish is lengthened by adding naturally occurring substances to the feed of the fish, such as, immune enhancing ingredients like beta-glucans and/or small amounts of phytohormones like auxin or gibberellic acid. All toxicology studies conducted to this point indicate abscisic acid, is a safe, nontoxic substance.

Here, Applicants surprisingly discovered that abscisic acid and/or salts thereof, have health and feed benefits for livestock and aquaculture.

### SUMMARY OF THE INVENTION

The present invention generally relates to compositions comprising abscisic acid and/or salts thereof (collectively referred to as "ABA" herein), of which (S)-(+)-abscisic acid is one enantiomer (hereinafter referred to as "S-ABA").

The present invention is directed to methods of increasing feed efficiency comprising administering to livestock or fish an effective amount of ABA.

It is contemplated that another embodiment of the present invention is directed to methods of increasing weight gain of livestock and fish comprising administering to an animal an effective amount of ABA.

It is also contemplated that another embodiment of the present invention is directed to methods of decreasing the time to market of livestock and fish comprising administering to livestock or fish an effective amount of ABA.

It is also contemplated that a different embodiment of the present invention is directed to methods of increasing the meat quantity of livestock and fish comprising administering to livestock or fish an effective amount of ABA.

Compositions of the present invention generally comprise ABA. Other components which enhance the biological activity of the ABA may optionally be included.

The disclosed embodiments are simply exemplary embodiments of the inventive concepts disclosed herein and should not be considered as limiting, unless the claims expressly state otherwise.

### DETAILED DESCRIPTION OF THE INVENTION

The present invention is specifically directed to methods for administering compositions comprising ABA, of which (S)-(+)-abscisic acid is one enantiomer to treat various animal ailments, as well as being used as feed for animals and animal breeders.

### Definitions

The term "Abscisic acid" as used herein shall include salts (collectively referred to as "ABA"). S-ABA is the preferred compound of the compositions and uses herein and has the structure as follows:

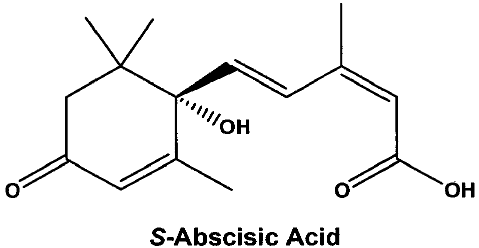

Compositions and methods of the inventions encompass the racemic mixtures, enol forms, solvated and unsolvated forms and pharmaceutically acceptable salts, of the described abscisic acids. Examples of suitable salts that can be used include inorganic salts such as the ammonium, lithium, sodium, potassium, magnesium, and potassium salts and organic amine salts such as the triethanolamine, dimethanolamine and ethanolamine salts. In one embodiment, the organic amine salt is the triethanolamine salt. In another embodiment, the organic amine salt is the dimethylethanolamine salt. In yet another embodiment, the organic amine salt is the ethanolamine salt. These examples of salts are not limiting as other salts may also be suitable for use in the present invention. One presently preferred salt is the ammonium salt. Other preferred salts are the sodium and potassium salts. The salts may be prepared by contacting the acid form with a sufficient amount of the desired base to produce a salt in the conventional manner. The free acid forms may be regenerated by treating the salt with a suitable dilute aqueous acid solution such as dilute aqueous sulfuric, hydrochloric or phosphoric acid. The free acid forms differ from their respective salt forms somewhat in certain physical properties, such as their solubilities in polar solvents, but the salts are equivalent to their respective free acid forms for purposes of the invention. (See, for example S. M. Berge, et al., "Pharmaceutical Salts," J. Pharm. Sci., 66: 1-19 (1977)).

The term "livestock and fish" refer to any livestock or fish, or offspring of livestock or fish. The term "offspring" refers to progeny or descendants of livestock or fish, and includes born progeny, fetuses and embryos. "Livestock" shall include poultry, including chickens, which includes boilers and layers and male and female breeding stock, geese, duck, turkey, pheasant, cornish hens, swine, cattle, which includes beef and dairy production, sheep, and goats. "Fish" shall include catfish, carp, tilapia, trout, crayfish, shrimp, lobster, crab, aquatic mammals, salmon, and white fish.

The term "administering" or "administration" includes any means for introducing the ABA of the invention, into the body.

The term "composition" includes a product comprising ABA (and in the specified amounts, if indicated), including products with exogenous or up-regulated ABA, as well as any product which results, directly or indirectly, from combination of ABA with specified ingredients in the specified amounts.

The term "effective amount" means an amount of a compound that, when administered to livestock or fish for attaining a desired result, is sufficient to effect such desired result. The "effective amount" will vary depending on the compound, the health benefit desired, the result desired, the age and relative health of the livestock or fish, the route and form of administration, the judgment of the attending medical or veterinary practitioner, breeder, trainer, or person attending or caring for the livestock or fish, and other factors. The amount of ABA that is "effective" will vary from composition to composition, depending on the particular use, the particular ABA, salts, and the like. Thus, it is not always possible to specify an exact "effective amount." However, an appropriate "effective amount" in any individual case may be determined by one of ordinary skill in the art using routine experimentation.

The term "Metric Ton" (MT) refers to 2,205 pounds or 1,000 kg. The term "ton", also known as a "short ton", refers to 2,000 pounds or 907 kg.

As used herein, all numerical values relating to amounts, weight percentages and the like are defined as "about" or "approximately" each particular value, namely, plus or minus 10%. For example, the phrase "at least 5% by weight" is to be understood as "at least 4.5% to 5.5% by weight." Therefore, amounts within 10% of the claimed values are encompassed by the scope of the claims.

### Composition Form for Administration

The compositions of the present invention may be incorporated with compound feed, or commercial pelleted food produced in a feed mill and fed to domestic livestock and fish. In varying embodiments, the compositions of the present invention may be incorporated with fodder, or food given to domestic livestock including plants cut and carried to them. In other varying embodiments, the compositions of the present invention may be incorporated with forage, or growing plants eaten by domestic livestock. It is within a skill in the art to formulate the compositions with foodstuffs for oral consumption.

In a different embodiment, the compositions of the present invention may be formulated in a liquid composition for oral consumption. It is within a skill in the art to formulate the compositions in liquid compositions for oral consumption.

In yet another embodiment, the compositions of the present invention may be added to water used for livestock or fish production. In yet another varying embodiment, the composition of the present invention is added to an aquaculture system used to grow fish, aquatic mammals, or crustaceans.

An embodiment of the present invention is directed to methods of increasing weight gain of livestock and fish comprising administering to livestock and fish an effective amount of ABA.

It is contemplated that another embodiment of the present invention is directed to methods of decreasing the time to market of livestock and fish comprising administering to livestock and fish an effective amount of ABA. It is also contemplated that a different embodiment of the present invention is directed to methods of increasing the meat quantity of livestock and fish comprising administering to livestock and fish an effective amount of ABA.

A preferred range of an effective amount of ABA for the various methods is from about 1 gram/MT of feed to about 2000 grams/MT of feed. A more preferred range of ABA is from about 5 grams/MT of feed to about 500 grams/MT of feed. A person having ordinary skill in the art would be able to adjust these ranges for various uses like aquaculture applications without undue experimentation.

### Animal Feed

Animal feed for feeding poultry includes protein, fat, fiber, calcium, and phosphorous. A preferred feed would include corn and/or wheat, soybean meal, fat, animal by-product, meat and bone meal, and vitamins and minerals.

When used in poultry feed, ABA can be initially mixed into a premix. The term "premix" is intended to mean a feed composition that is prepared as an initial mix containing the active ingredient and, for example, a carrier, and is then blended into the final feed. In the present invention, it is generally suggested that, for ease of calculation and use, the premix is blended with about one metric ton (MT) of conventional feed, with the result that the necessary dosage requirements of the ABA are provided to the animals. When preparing one metric ton (MT) of finished feed, the premix of the invention preferably comprises about 1 to 2000, and more preferably, about 5 to 500, grams of ABA. Carriers for use in a premix are well known by those having skill in the art, and appropriate concentrations can be readily determined.

The ABA may be added to the carrier as a dry powder or as a liquid solution or suspension. When added as a liquid, the ABA may be dissolved or suspended in a liquid with stirring at room temperature. Such liquid may be water or a suitable solvent or another product used for animal feed that is already in liquid form. Because of ABA's liquid solubility characteristics, it may form a suspension. A predetermined amount of ABA is then added to the conventional premix, and will not overly wet it. Once the premix is prepared, the premix is then added to the final feed, preferably at a rate in the range of one quarter of a pound (lb.) to five pounds (lbs.) of premix to one metric ton (MT) of feed, to supply daily requirements of the ABA for the poultry.

The ABA solid or solution or liquid suspension can be added directly to the premix material, and then mixed. Mixing can be accomplished by any known means, such as by a standard horizontal or vertical blender. Mixing time will again vary depending upon the particular ingredients of the premix, and can take as long as is necessary to assure that the ingredients are thoroughly mixed.

The premix is then incorporated into the feed to be fed to the poultry. In a more preferred embodiment, ABA is blended with the carrier to form the premix, and the premix is directly blended into the final feed. While there is no evidence that use of the higher amounts would cause any toxicity problems in treated poultry, it will affect cost considerations. Because the premix generally will be added to one-ton charges of feed (as is common in the industry), the correspondence between one gram of ABA added to the premix yields about 1 ppm concentration of ABA in the feed. Thus, 5 grams of ABA added to one pound of premix, which in turn is added to one metric ton (MT) of feed, yields about a 5 ppm effective ABA concentration.

While the ABA can be mixed with a premix material prior to incorporation into the finished feed, the appropriate amount of ABA may be directly blended into or sprayed upon the feed. The preferred additive range of ABA in finished feed, whether added directly or via a premix, is about 1 to 2000 grams per metric ton (MT) of feed; more preferably about 5 to 500 grams per metric ton (MT) of feed.

Feed is conventionally prepared in a large bin or mixer in which the feed ingredients are added in descending weight order according to their prevalence in the ultimate feed mixture. Thus, cracked or ground grain would be the primary ingredient. Minor ingredients are then added. Micro-ingredients are added last. These include vitamins, drugs, growth promoters, antibiotics, and, in the present case, ABA. Thus, ABA can be one of the micro-ingredients and is added to the feed in the final blending step. The feed is blended for conventional time periods.

The feed comprising the ABA is fed to livestock and fish in standard form, such as a mash, crumble or pellet, and at standard feed dosage ranges and rates.

Another embodiment of the present invention is liquid compositions that can be prepared as either ready-to-use dilutions or dilutable concentrates. The embodiment of the present invention can be a solution containing from 0.5% to as much as 50% by weight of ABA. The dilutable concentrates can be diluted into water directly to a final application concentration or to any intermediate dilution, without risk of precipitation of the active ingredient. The aqueous formulations according to one embodiment of the present invention are inexpensive to manufacture, safe to handle and use, and the ABA active ingredient is stable under storage and shipping conditions. A person having ordinary skill in the art would be able to determine how to prepare the final aqueous solution concentration for direct application to animals without undue experimentation, without any chance of causing precipitation of the active ingredient, and without long and laborious stirring to bring the active ingredient into solution.

Another embodiment of the present invention is an ABA water solution that serves as a drinking source of water for the livestock and fish. Such supplemented water solution could be prepared by dissolving dry powder ABA in drinking water or by using a liquid solution or suspension concentrate of ABA. The preferred additive range of ABA in drinking water is about 1 to 2000 parts per million (ppm) or about 1 to 2000 milligrams of ABA per liter of water; more preferably about 5 to 500 parts per million (ppm) or about 5 to 500 milligrams of ABA per liter of water. A person having ordinary skill in the art would be able to determine how to prepare the final aqueous solution for direct application to livestock and fish without undue experimentation, without any chance of causing precipitation of the active ingredient, and without long and laborious stirring to bring the active ingredient into solution.

in another embodiment of the present invention ABA can be applied directly onto animal feed once it has been prepared. For example, as can be the practice with some enzymes, the ABA can be applied directly to the finished feed. In a preferred embodiment, an aqueous solution of ABA is sprayed onto the finished feed in its final form, such as a pellet, prior to delivering the feed to the livestock and fish.

The advantageous properties of this invention can be observed by reference to the following examples that illustrate the invention. These examples are provided for the purposes of illustration and are not intended to limit the scope of the present invention.

The following examples are intended to illustrate the present invention and to teach one of ordinary skill in the art how to make and use the invention. They are not intended to limit the invention or its protection in any way.

### EXAMPLE 1

Male Cobb 500 chickens were fed different levels of S-ABA supplemented into starter, grower, and finisher feed diets for their entire 42 day commercial life cycle. On day 43, samples of chickens (sometimes referred to as "birds") from each treatment were processed.

### A. BASAL AND EXPERIMENTAL DIETS

The starter, grower, and finisher basal diets represented standard commerical broiler diets. The starter and grower diets contained Salinomycin (a coccidiostat) at 40 grams/ton. The basal diets and final treatment diets were prepared using a 500 or 4,000 pound capacity vertical mixer or a 14,000 pound capacity horizontal mixer. After correcting for percent purity of S-ABA in the S-ABA dry powder, S-ABA was added to the basal diets at concentrations of 5, 50, or 500 ppm (4.6, 46, or 455 grams of S-ABA/ton). Control treatments included (1) commercial diet only and (2) commercial diet supplemented with antibiotics (bacitracin methylene disalicylate (BMD), and STAFAC® virginiamycin (hereinafter "Stafac"). STAFAC is a registered trademark of Phibro Animal Health Corporation). All feed diets were pelleted at ∼65°C or below.

### B. TEST GROUPS

A 90-pen test facility was divided into 10 blocks containing 9 pens per block. Treatments were assigned to the pens using a complete randomized block design. Birds were assigned to the pens randomly. Specific treatment groups were as follows:

| **Treatment** | **Diet** | **Diet Code** | **Litter** | **No. of Birds per Pen*** | **No. of Pens per Treatment** | **No. of Birds per Treatment** | **∼Feed Consumption (kg)** |
|---|---|---|---|---|---|---|---|
| 1 | Commercial feed diet | A | Clean | 18 | 10 | 180 | 1,209 |
| 2 | S-ABA 5 ppm | B | Clean | 18 | 10 | 180 | 1,209 |
| 3 | S-ABA 50 ppm | C | Clean | 18 | 10 | 180 | 1,209 |
| 4 | S-ABA 500 ppm | D | Clean | 18 | 10 | 180 | 1,209 |
| | | | | | | | |
| 5 | Commercial feed diet | A | Dirty | 18 | 10 | 180 | 1,209 |
| 6 | S-ABA 5 ppm | B | Dirty | 18 | 10 | 180 | 1,209 |
| 7 | S-ABA 50 ppm | C | Dirty | 18 | 10 | 180 | 1,209 |
| 8 | S-ABA 500 ppm | D | Dirty | 18 | 10 | 180 | 1,209 |
| 9 | BMD 50 grams/ton in starter feed diet, 25 grams/ton in grower feed diet, Stafac 10 grams/ton in finisher feed diet | E | Dirty | 18 | 10 | 180 | 1,209 |
| Total | | | | | 90 | 1,620 | 10,881 |

| | | | | | | | |
|---|---|---|---|---|---|---|---|
| * Two (2) extra chicks were placed in each pen on day 0 (20 birds/pen). At 7 days of age the birds in each pen were counted and bird counts in each pen were adjusted to 18 birds/pen. Extras were weighed, recorded, and removed. | | | | | | | |

### C. MANAGEMENT

### Vaccinations and Treatments:

Birds were vaccinated for Mareks at the hatchery. Birds were vaccinated upon receipt for the study (day 0) for Newcastle and Infectious Bronchitis via spray cabinet. No other vaccinations or treatments, except what is provided in the diets were administered during the study.

### Water:

Water was provided ad libitum throughout the study via one automatic four-nipple drinker in each pen. Drinkers were checked twice daily to assure a constant and clean water supply to the birds.

### Feed:

Feed was provided ad libitum throughout the study via one handing, ~17-inch diameter tube feeder per pen. Chick feeder trays were placed in each pen for approximately the first 4 days. Feed added and removed from pens was weighed and recorded from day 0 to day 42. Diet changes were conducted at the same time for all pens. The feeding period for each diet was as shown below:

| **Diet** | **Form** | **Feeding Period (Days)** |
|---|---|---|
| Starter | Crumble | 0-14 |
| Grower | Pellet | 14-28 |
| Finisher | Pellet | 28-42 |

### Mortality:

Starting on study day 0, any bird found dead or needing to be removed and sacrificed was weighed and necropsied. The probable cause of death and necropsy findings were recorded.

### Body Weights and Feed Intake:

Birds were weighed by pen at 0, 7, 14, 21, 28, 35, and 42 days of age. The feed remaining in each pen was weighed and recorded on study day 0, 7, 14, 21, 28, 35, and 42 days of age. The feed intake during days 0-7, 0-14, 0-21, 0-28, 0-35, and 0-42 was calculated.

### Weight Gains and Feed Conversion:

Performance data was summarized by average weight per bird at each body weights measurement period. The average feed conversion was calculated for each respective body weight period using the total feed consumption in a pen divided by the total weight of surviving birds (i.e. days 0-7, 0-14, 0-21, etc.). Adjusted feed conversion was calculated using the total feed consumption in a pen divided by the total weight of surviving birds and weight of birds that died or were removed from that pen.

### Processing:

Four blocks (9 pens/block) were selected at random from each treatment. On day 42 all birds in the selected blocks were individually wing banded with a unique number. Prior to processing the birds were fasted for approximately 12 hours. Immediately after processing on day 43, digestive tract weights and Bursa of Fabricius organ weights were measured.

### D. RESULTS

TABLE 1 shows that 5 ppm S-ABA added to commercial feed diets had significantly (P= 0.05) lower bird mortality compared to the control treatments, including the commercial feed diet containing the bacitracin methylene disalicylate and Stafac antibiotics.

**TABLE 1**

| **Cumulative Bird Mortality After 42 Days** | | |
|---|---|---|
| **Treatment*** | **Lived** | **Died** |
| Commercial feed diet (control 1) | 194 | 6 |
| Commercial feed diet plus BMD, Stafac (control 2) | 194 | 6 |
| Commercial feed diet plus 5 ppm S-ABA | 200 | 0 |

| | | |
|---|---|---|
| *Dirty litter conditions | | |

The greater level of bird health in response to S-ABA, as shown in TABLE 1 above, was observed under dirty litter conditions, which represents a challenging and stressful living environment for the birds.

TABLE 2 shows that 5 ppm S-ABA added to commercial feed increased bird growth (weights) compared to the commercial feed diet (control 1). The addition of 5 ppm S-ABA to the commercial feed diet also reduced feed conversion ratios by 1-4 points. Feed conversion ratios that were corrected for the number of surviving birds (i.e., adjusted feed conversion ratios) were lower by 1-2 points.

**TABLE 2**

| **Bird Weights and Feed Conversion Ratios*** | | | | | | |
|---|---|---|---|---|---|---|
| | Means | Means | Means | Means | Means | Means |
| **Bird Weight (kg)** | **Day 7** | **Day 14** | **Day 21** | **Day 28** | **Day 35** | **Day 42** |
| Commercial feed diet (control 1) | 0.1427 | 0.3763 | 0.7588 | 1.3785 | 2.0704 | 2.7010 |
| versus | vs. | vs. | vs. | vs. | vs. | vs. |
| Commercial feed diet plus 5 ppm S-ABA | 0.1445 | 0.3873 | 0.7899 | 1.4236 | 2.0919 | 2.7565 |

| **Feed/Gain Ratio** | **Day 7** | **Day 14** | **Day 21** | **Day 28** | **Day 35** | **Day 42** |
|---|---|---|---|---|---|---|
| Commercial feed diet (control 1) | 1.4505 | 1.3544 | 1.4245 | 1.4915 | 1.5864 | 1.6818 |
| versus | vs. | vs. | vs. | VS. | vs. | vs. |
| Commercial feed diet plus 5 ppm S-ABA | 1.4093 | 1.3400 | 1.4137 | 1.4584 | 1.5731 | 1.6861 |

| **Adjusted Feed/Gain Ratio** | **Day 7** | **Day 14** | **Day 21** | **Day 28** | **Day 35** | **Day 42** |
|---|---|---|---|---|---|---|
| Commercial feed diet (control 1) | 1.4218 | 1.3009 | 1.4028 | 1.4617 | 1.5652 | 1.6649 |
| versus | vs. | vs. | vs. | vs. | vs. | vs. |
| Commercial feed diet plus 5 ppm S-ABA | 1.4093 | 1.2854 | 1.3842 | 1.4517 | 1.5610 | 1.6691 |

| | | | | | | |
|---|---|---|---|---|---|---|
| *Dirty litter conditions | | | | | | |

Increased bird growth, as evidenced by the greater weights shown in TABLE 2, suggests that the addition of S-ABA to commercial feed can lead to faster growth and bigger birds. This response to S-ABA could prove useful by reducing the number of days to reach a given target bird size (weight). The reduction in feed conversion ratios means the addition of S-ABA to animal feed can lower the cost to produce a given unit of meat (e.g., a pound of chicken breast meat).

TABLE 3 shows that S-ABA in the feed diet increases the weight of digestive tracts compared to the commercial feed diets (control 1 and control 2).

**TABLE 3**

| **Digestive Tract Weights** | |
|---|---|
| **Treatment*** | **Mean Weight (Grams)** |
| Commercial feed diet plus 500 ppm S-ABA | 88.4 |
| Commercial feed diet plus 5 ppm S-ABA | 86.7 |
| Commercial feed diet plus 50 ppm S-ABA | 86.6 |
| Commercial feed diet plus BMD, Stafac (control 2) | 85.9 |
| Commercial feed diet (control 1) | 84.4 |

| | |
|---|---|
| *Dirty litter conditions | |

Increased digestive tract weights, as shown in TABLE 3 above, may be directly related to better overall health. Further, data in TABLE 3 suggest that S-ABA may also reduce or eliminate the need for antibiotics in feed.

With these data (TABLES 1-3), one can see that S-ABA improves the health and growth of chickens and increases feed efficiencies so that producers can save costs.

TABLE 4 shows the economic benefit of improved health and growth of broiler chickens and increased feeding efficiency resulting from the addition of S-ABA into commercial feed.

**TABLE 4**

| **Profit Value per 1,000 Birds Started*** | | |
|---|---|---|
| **Base Feed Cost ($/ton)** | **Commercial feed diet (control 1) versus Commercial feed diet plus 5 ppm S-ABA** | **Commercial feed diet plus BMD, Stafac (control 2) versus Commercial feed diet plus 5 ppm S-ABA** |
| $150 | $112.75 | $68.74 |
| $200 | $99.44 | $61.74 |
| $250 | $86.12 | $54.73 |
| $325 | $66.16 | $44.22 |

| | | |
|---|---|---|
| * See US patent 5,715, 185; also Poultry USA magazine, May 2003, pgs 34-40. | | |

The tabulated results in TABLE 4 show that by decreasing broiler chicken production costs through better health increased bird growth, and improved feed conversion efficiencies, the addition of ABA into commercial broiler feed can result in meaningful economic benefit to the industry. For instance, at a base feed cost of $250/ton when compared to the commercial feed diet (control 1), the addition of 5 ppm S-ABA to the diet added an incremental $86.12 in profit value per 1,000 birds started. Compared to the commercial feed diet plus BMD and Stafac (control 2), the addition of 5 ppm S-ABA to the diet increased the profit value per 1,000 birds started by $54.73.

### EXAMPLE 2

Male and female Cobb 500 chickens were fed different levels of (s) - (+) - abscisic acid (S-ABA) supplemented into starter, grower, and finisher feed diets for their entire 42 days commercial life cycle.

### A. BASAL AND EXPERIMENTAL DIETS

The starter, grower, and finisher basal diets represented standard commercial broiler diets. The starter and grower diets contained Salinomycin (a coccidiostat) at 40 grams/ton. The basal diets and final treatment diets were prepared using a 500 or 4,000 pound capacity vertical mixer or a 14,000 pound capacity horizontal mixer. After correcting for percent purity of S-ABA in the S-ABA dry powder, S-ABA was added to the basal diets at concentrations of 2.5, 5.0, or 7.5 ppm (2.3, 4.6, or 6.8 grams of S-ABA/ton). Control treatments included (1) commercial diet only and (2) commercial diet supplemented with antibiotics (bacitracin methylene disalicylate and Stafac). All feed diets were pelleted at ∼65°C or below.

### B. TEST GROUPS

A 108-pen test facility was divided into 18 blocks containing 6 pens per block. Treatments were assigned to the pens using a complete randomized block design. Birds were assigned to the pens randomly. Specific treatment groups were as follows:

| **Treatment** | **Diet** | **Diet Code** | **Sex** | **No. of Birds per Pen*** | **No. of Pens per Treatment** | **No. of Birds per Treatment** |
|---|---|---|---|---|---|---|
| 1 | Commercial feed diet | A | F | 18 | 18 | 324 |
| 2 | BMD 50 grams/ton in starter feed diet, 25 grams/ton in grower feed diet, Stafac 10 grams/ton in finisher | B | F | 18 | 18 | 324 |
| 3 | S-ABA 2.5 ppm | C | F | 18 | 18 | 324 |
| 4 | S-ABA 5.0 ppm | D | F | 18 | 18 | 324 |
| 5 | S-ABA 7.5 ppm | E | F | 18 | 18 | 324 |
| 6 | S-ABA 5.0 ppm | D | F | 18 | 18 | 324 |
| | | | | | | |
| 7 | Commercial feed diet | A | M | 18 | 18 | 324 |
| 8 | BMD 50 grams/ton in starter feed diet, 25 grams/ton in grower feed diet, Stafac 10 grams/ton in finisher | B | M | 18 | 18 | 324 |
| 9 | S-ABA 2.5 ppm | C | M | 18 | 18 | 324 |
| 10 | S-ABA 5.0 ppm | D | M | 18 | 18 | 324 |
| 11 | S-ABA 7.5 ppm | E | M | 18 | 18 | 324 |
| 12 | S-ABA 5.0 ppm | D | M | 18 | 18 | 324 |
| Total | | | | | 216 | 3,888 |

| | | | | | | |
|---|---|---|---|---|---|---|
| * Nine (9) extra chicks were placed in each pen on day 0 (27 birds/pen). At 21 days of age, after pen weights, the birds were counted and adjusted by randomly selecting and removing the appropriate number of birds to achieve 18 birds/pen. Composite weights were taken on the removed birds and then subtracted from the initial 21 day weights. | | | | | | |

### C. MANAGEMENT

### Vaccinations and Treatments:

Birds were vaccinated for Mareks at the hatchery. Birds were vaccinated upon receipt for the study (day 0) for Newcastle and Infectious Bronchitis via spray cabinet. No other vaccinations or treatments, except what is provided in the diets were administered during the study.

### Water:

Water was provided ad libitum throughout the study via one automatic four-nipple drinker in each pen. Drinkers were checked twice daily to assure a constant and clean water supply to the birds.

### Feed:

Feed was provided ad libitum throughout the study via one handing, ∼17-inch diameter tube feeder per pen. Chick feeder trays were placed in each pen for approximately the first 4 days. Feed added and removed from pens was weighed and recorded from day 0 to day 42. Diet changes were conducted at the same time for all pens. The feeding period for each diet was as shown below:

| **Diet** | **Form** | **Feeding Period (Days)** |
|---|---|---|
| Starter | Crumble | 0-21 |
| Grower | Pellet | 21-35 |
| Finisher | Pellet | 35-42 |

### Mortality:

Starting on study day 0, any bird found dead or needing to be removed and sacrificed was weighed and necropsied. The probable cause of death and necropsy findings were recorded.

### Body Weights and Feed Intake:

Birds were weighed by pen at 0, 7, 14, 21, 28, 35, and 42 days of age. The feed remaining in each pen was weighed and recorded on study day 0, 7, 14, 21, 28, 35, and 42 days of age. The feed intake during days 0-7, 0-14, 0-21, 0-28, 0-35, and 0-42 was calculated.

### Weight Gains and Feed Conversion:

Performance data was summarized by average weight per bird at each body weights measurement period. The average feed conversion was calculated for each respective body weight period using the total feed consumption in a pen divided by the total weight of surviving birds (i.e. days 0-7, 0-14, 0-21, etc.). Adjusted feed conversion was calculated using the total feed consumption in a pen divided by the total weight of surviving birds and weight of birds that died or were removed from that pen.

### D. RESULTS

TABLE 5 shows that ABA added to commercial feed diets had higher survival rates compared to the commercial feed diet containing the bacitracin methylene disalicylate and Stafac antibiotics.

**TABLE 5**

| **Cumulative Bird Mortality After 42 Days** | | |
|---|---|---|
| Treatment* | Sex | Mortality |
| Commercial feed diet | Female | 1.8% |
| BMD 50 grams/ton in starter feed diet, 25 grams/ton in grower feed diet, Stafac 10 grams/ton in finisher | Female | 3.5% |
| S-ABA 2.5 ppm | Female | 2.4% |
| S-ABA 5.0 ppm | Female | 0.4% |
| S-ABA 7.5 ppm | Female | 2.2% |
| S-ABA 5.0 ppm | Female | 1.9% |
| Commercial feed diet | Male | 3.0% |
| BMD 50 grams/ton in starter feed diet, 25 grams/ton in grower feed diet, Stafac 10 grams/ton in finisher | Male | 4.5% |
| S-ABA 2.5 ppm | Male | 4.1% |
| S-ABA 5.0 ppm | Male | 2.9% |
| S-ABA 7.5 ppm | Male | 3.8% |
| S-ABA 5.0 ppm | Male | 3.5% |

| | | |
|---|---|---|
| * Dirty litter conditions | | |

The greater level of bird survival in the S-ABA treatments, as shown in TABLE 5 above, was observed under dirty litter conditions, which represents a more challenging and stressful living environment for the birds. This suggests that additional S-ABA in the feed may help improve bird health.

With these results (TABLES 1-5), one can see that S-ABA improves the health and growth of chickens and increases feeding efficiency so that producers can save costs. Taking these various parameters into consideration, the benefits of supplementing animal feed with additional S-ABA means greater economic benefit to animal producers by improving operating efficiencies during bird production and higher meat yield upon processing.

## Claims

1. A method of increasing feed efficiency comprising administering to livestock or fish an effective amount of abscisic acid and/or salts thereof.

2. A method of increasing weight gain of livestock or fish, comprising administering to livestock or fish an effective amount of abscisic acid and/or salts thereof.

3. A method of decreasing the time to market of livestock or fish, comprising administering to livestock or fish an effective amount of abscisic acid and/or salts thereof.

4. A method of increasing the meat quantity of livestock or fish, comprising administering to livestock or fish an effective amount of abscisic acid and/or salts thereof.

5. The method of any one of claims 1 to 4, wherein the effective amount is from 1 gram ± 10% per metric ton of feed to 2000 grams ± 10% per metric ton of feed.

6. The method of any one of claims 1 to 4, wherein the effective amount is from 5 grams ± 10% per metric ton of feed to 500 grams ± 10% per metric ton of feed.

7. The method of any one of claims 1 to 4, wherein the abscisic acid and/or salts thereof is administered as a liquid composition to the livestock.

8. The method of any one of claims 1 to 4, wherein the abscisic acid and/or salts thereof is administered as a water solution that serves as a drinking source to the livestock.

9. The method of claim 8, wherein the effective amount is from 1 ± 10% to 2000 ± 10% parts per million of abscisic acid and/or salts thereof.

10. The method of claim 8, wherein the effective amount is from 5 ± 10% to 500 ± 10% parts per million of abscisic acid and/or salts thereof.

## Patentansprüche

1. Verfahren zum Vergrößern der Futtereffizienz, das das Verabreichen einer effektiven Menge von Abscisinsäure und/oder Salzen davon an Nutztiere oder Fische beinhaltet.

2. Verfahren zum Vergrößern der Gewichtszunahme von Nutztieren oder Fischen, das das Verabreichen einer effektiven Menge von Abscisinsäure und/oder Salzen davon an Nutztiere oder Fische beinhaltet.

3. Verfahren zum Verringern der Zeit bis zur Marktreife von Nutztieren oder Fischen, das das Verabreichen einer effektiven Menge von Abscisinsäure und/oder Salzen davon an Nutztiere oder Fische beinhaltet.

4. Verfahren zum Vergrößern der Fleischmenge von Nutztieren oder Fischen, das das Verabreichen einer effektiven Menge von Abscisinsäure und/oder Salzen davon an Nutztiere oder Fische beinhaltet.

5. Verfahren gemäß einem der Ansprüche 1 bis 4, wobei die effektive Menge von 1 Gramm ± 10 % pro metrischer Tonne Futter bis 2000 Gramm ± 10% pro metrischer Tonne Futter reicht.

6. Verfahren gemäß einem der Ansprüche 1 bis 4, wobei die effektive Menge von 5 Gramm ± 10 % pro metrischer Tonne Futter bis 500 Gramm ± 10% pro metrischer Tonne Futter reicht.

7. Verfahren gemäß einem der Ansprüche 1 bis 4, wobei die Abscisinsäure und/oder Salze davon als flüssige Zusammensetzung an die Nutztiere verabreicht werden.

8. Verfahren gemäß einem der Ansprüche 1 bis 4, wobei die Abscisinsäure und/oder Salze davon als Wasserlösung, die als Trinkquelle dient, an die Nutztiere verabreicht werden.

9. Verfahren gemäß Anspruch 8, wobei effektive Menge von 1 ± 10 % bis 2000 ± 10 % Teile pro Million Abscisinsäure und/oder Salze davon reicht.

10. Verfahren gemäß Anspruch 8, wobei effektive Menge von 5 ± 10 % bis 500 ± 10 % Teile pro Million Abscisinsäure und/oder Salze davon reicht.

## Revendications

1. Procédé permettant d'augmenter l'efficience alimentaire, comprenant l'administration à un cheptel ou à un poisson d'une quantité efficace d'acide abscissique et/ou de sels de celui-ci.

2. Procédé permettant d'accroître le gain de poids d'un cheptel ou d'un poisson, comprenant l'administration à un cheptel ou à un poisson d'une quantité efficace d'acide abscissique et/ou de sels de celui-ci.

3. Procédé permettant de diminuer le délai de mise sur le marché d'un cheptel ou d'un poisson, comprenant l'administration à un cheptel ou à un poisson d'une quantité efficace d'acide abscissique et/ou de sels de celui-ci.

4. Procédé permettant d'augmenter la quantité de viande d'un cheptel ou d'un poisson, comprenant l'administration à un cheptel ou à un poisson d'une quantité efficace d'acide abscissique et/ou de sels de celui-ci.

5. Procédé selon l'une quelconque des revendications 1 à 4, ladite quantité efficace représentant de 1 gramme ± 10 % par tonne métrique de nourriture à 2000 grammes ± 10 % par tonne métrique de nourriture.

6. Procédé selon l'une quelconque des revendications 1 à 4, ladite quantité efficace représentant de 5 grammes ± 10 % par tonne métrique de nourriture à 500 grammes ± 10 % par tonne métrique de nourriture.

7. Procédé selon l'une quelconque des revendications 1 à 4, ledit acide abscissique et/ou lesdits sels de celui-ci étant administrés sous la forme d'une composition liquide au cheptel.

8. Procédé selon l'une quelconque des revendications 1 à 4, ledit acide abscissique et/ou lesdits sels de celui-ci étant administrés sous la forme d'une solution aqueuse qui sert de source potable au cheptel.

9. Procédé selon la revendication 8, ladite quantité efficace représentant de 1 ± 10 % à 2000 ± 10 % parties par million d'acide abscissique et/ou de sels de celui-ci.

10. Procédé selon la revendication 8, ladite quantité efficace représentant de 5 ± 10 % à 500 ± 10 % parties par million d'acide abscissique et/ou de sels de celui-ci.
